Europäisches Patentamt

(19) European Patent Office

Office européen des brevets

(11) Publication number: **0 161 847**
A2

# (12) EUROPEAN PATENT APPLICATION

(21) Application number: 85302936.1

(22) Date of filing: 25.04.85

(51) Int. Cl.⁴: **A 01 J 7/00,** G 01 N 1/10,
G 01 F 1/00, G 01 F 5/00

(30) Priority: 12.05.84 NZ 207822

(43) Date of publication of application: 21.11.85
Bulletin 85/47

(84) Designated Contracting States: AT BE CH DE FR GB IT
LI LU NL SE

(71) Applicant: ANGLIAN LIMITED, c/o Cook Island Trust
Corporation Limited, Rarotonga Cook Islands (NZ)

(72) Inventor: Hartstone, John Lewis, 3 Pycroft Place, St.
Heliers Auckland (NZ)

(74) Representative: Leale, Robin George et al, FRANK B.
DEHN & CO. Imperial House 15-19 Kingsway, London
WC2B 6UZ (GB)

(54) Metering arrangement.

(57) The present invention relates to a liquid metering arrangement including a liquid meter (1) and at least one sample chamber (4) in communication therewith; transfer means (3) being provided to allow for the passage of liquid from said meter (1) into said sample chamber (4); valve means being provided in association with said transfer means (3); an air inlet (6) being provided adjacent an upper portion of the sample chamber (4) and having valve means (7) associated therewith; a liquid outlet pipe (14) being provided within said sample chamber (4) and extending out of an upper portion thereof, so as to be connectable to a sub-sampler (12); the arrangement being such that on an amount of liquid being present within said sample chamber (4), said valving (7) associated with said air inlet (6) into said sample chamber (4) is opened allowing an inlet of air, thereby causing closure of said valving associated with the liquid transfer means (3) and further causing liquid within the sample chamber (4) to pass through the liquid outlet pipe (14) to said sub-sampler (12).

There is also provided a sample arrangement for obtaining a sample of liquid from a liquid flow, including a housing (60); liquid inlet and outlet means (20, 21) being provided; a liquid flow line (23) extending therebetween and within said housing (60); a chamber (30) being provided and adapted so as to releasably engage a sample vial or container (40); said liquid flow line (23) being in communication with said chamber (30) such that a proportion of liquid passing through said liquid flow line (23) is able to pass into said chamber (40).

-1-

"METERING ARRANGEMENT"

This invention relates to an improved arrangement for the metering of liquids, such as for example milk, water and the like. However, the invention has particular (although not exclusive) application to the metering of milk.

It has been known up until this time, to provide liquid metering means in association with milking machines and milking arrangements by which milk is drawn from cows. Up until this time, cups of milking machines are placed over the teats of the cow, and vacuum applied, so that the milk is automatically transferred from the teats of the cows, through appropriate feedlines and the like, to milk supply means. In most countries throughout the world, it is necessary that samples of the milk be continuously taken, for the purposes of maintaining standards, checking hygiene, and generally maintaining and metering required standards. For this purpose, metering means have been provided for obtaining a sample of milk passing from cows to a milk storage tank, and samples obtained thereby are passed into an appropriate container or vial for subsequent testing. It will be appreciated that it is always highly desirable that the sample obtained is at least reasonably representative of and proportional relative to, the supply of milk being obtained from the cow.

By way of example, such a metering arrangement is disclosed in United States patent specification No. 3,349,617.

0161847

While the arrangement disclosed in United States patent specification No. 3,349,617 has proven to be particularly satisfactory and commercially successful, it is desirable to provide additions, alternatives and modifications thereto, especially having regard to the requirements of the dairy industry and various governmental authorities throughout the world, in relation to milk testing, sample testing and the like.

While the subject matter of the present application is described by way of example only, with reference to use with a metering arrangement such as that disclosed in United States patent specification 3,349,617, it should be appreciated that this is by way of example only. In addition, while the specification is described by way of example only, with reference to obtaining milk samples, it should be appreciated that the present subject matter could be used for obtaining samples of any other desired liquid.

### BRIEF SUMMARY OF THE PRESENT INVENTION

According to one aspect of this invention there is provided a liquid metering arrangement including a liquid meter; a liquid inlet line communicating with said meter; a liquid sample chamber being in communication with said meter; said liquid inlet line and said sample chamber being connected to allow for the flow of liquid therebetween; valve means being provided in association with said connection, such that on movement of said valve to at least a first position, flow of liquid from the inlet line, through said connection and into the chamber, is substantially

avoided; movement of said valve to at least a second position allowing at least a proportion of liquid passing through the inlet line, to be diverted through said connection and into said chamber.

An arrangement as claimed in claim 1, wherein movement of said valve to at least a second position, allows an amount of liquid to continue to pass through said inlet line, to said meter.

An arrangement as claimed in claim 1 or claim 2, wherein the sample chamber is substantially elongate in formation, the meter being located and in communication therewith, adjacent an upper end thereof; the connection between said sample chamber and said inlet line being adjacent the bottom of said sample chamber.

According to a further aspect of this invention there is provided a liquid metering arrangement including a liquid meter and at least one sample chamber in communication therewith; transfer means being provided to allow for the passage of liquid from said meter into said sample chamber; valve means being provided in association with said transfer means, an air inlet being provided adjacent an upper portion of the sample chamber and having valve means associated therwith; a liquid outlet pipe being provided within said sample chamber, and extending out of an upper portion thereof, so as to be connectable to a sub-sampler; the arrangement being such that on an amount of liquid being present within said sample chamber, valving associated with said air inlet into said sample chamber is opened allowing an inlet of air, thereby causing closure of said valving associated with the liquid transfer means and further causing liquid within the sample chamber to pass through the liquid outlet pipe to said sub-sampler.

According to a further aspect of this invention there is provided a liquid metering arrangement including a liquid meter and at least one sample in communication therewith; liquid supply means leading to said meter, the arrangement being such that at least a proportion of said liquid passes into said chamber; a liquid inlet line leading from an outlet of said chamber to a sub-sampler, means being provided to cause the flow of liquid from said chamber through said inlet line and into said sub-sampler; said sub-sampler arrangement including a housing having an inlet and an outlet; said liquid inlet line being connected to said inlet; a liquid outlet line being connected to said outlet and thereafter extending to said meter so as to be in communication therewith; a liquid flow line being provided within said housing, and connecting said inlet and outlet; a chamber being provided within said housing and being in communication with said liquid flow line such that a proportion of liquid passing through said liquid flow line is able to pass into said chamber; said chamber being adapted so as to releasably engage and locate a sample vial or container.

An arrangement as claimed in claim 5 wherein said sub-sampler includes control means to restrict passage of liquid through said liquid flow line.

An arrangement as claimed in claim 5, wherein an air outlet leads from said chamber into said liquid flow line.

An arrangement as claimed, wherein said control means are provided substantially intermediate the inlet from said liquid flow line into said chamber, and said air outlet from said chamber into said liquid flow line.

An arrangement as claimed in claim 6, wherein the control means is mounted outwardly of said liquid flow line; actuating means being associated therewith so that on actuation thereof, restriction means is caused to move inwardly of said liquid flow line, so as to be capable of restricting the flow of liquid therethrough.

An arrangement as claimed in claim 9, wherein the liquid flow line is provided with at least one bore or recess therein; a substantia-ly flexible and resilient sleeve extending about at least part of said liquid flow line and over said bore or recess; said restriction means including an elongate plunger mounted ajacent and over said bore or recess; the arrangement being such that on actuation of said actuating means, said elongate plunger is caused to move substantially onwardly and axially against said flexible and resilient sleeve, and through said bore or recess, so as to form an at least partial restriction in said liquid flow line.

An arrangement as claimed in claim 9, wherein said actuating means is in the form of a rotatable grip member having a substantially central boss portion and a radially extending flange with a peripheral, downwardly depending skirt; said skirt being provided with a plurality of markings or calibrations thereon; at least one marking being provided on the body portion of said sub-sampler, at a position substantially aligned with the location of said elongate plunger.

An arrangement as claimed in claim5, wherein a tubular inlet line extends from said liquid flow line into said chamber at a position inwardly of said inlet member; a tubular air outlet line leading from said chamber into said flow line, at a position spaced apart inwardly from said outlet.

An arrangement as claimed in claim 6, wherein said air outlet line is in the form of an elongate tube extending from said chamber, through said liquid flow line and said liquid outlet line, so as to be in communication with said meter; the diameter of said tubular air outlet being less than the diameter of said liquid outlet line leading from the outlet of said housing to said meter.

A sample arrangement for obtaining a sample of liquid from a liquid flow, including a housing; liquid inlet and outlet means being provided; a liquid flow line extending therebetween and within said housing; a chamber being provided and adapted so as to releasably engage a sample vial or container; said liquid flow line being in communication with said chamber such that a proportion of liquid passing through said liquid flow line is able to pass into said chamber.

## BRIEF DESCRIPTION OF THE DRAWINGS

The present invention will now be described by way of example only and with reference to the accompanying drawings wherein:

Figure 1: is a general view of an arrangement according to one form of the present invention.

Figure 2: is a sectional view of a sub-sampler arrangement according to one form of the present invention.

### DETAILED DESCRIPTION OF THE PREFERRED EMBODIMENTS OF THE PREFERRED INVENTION

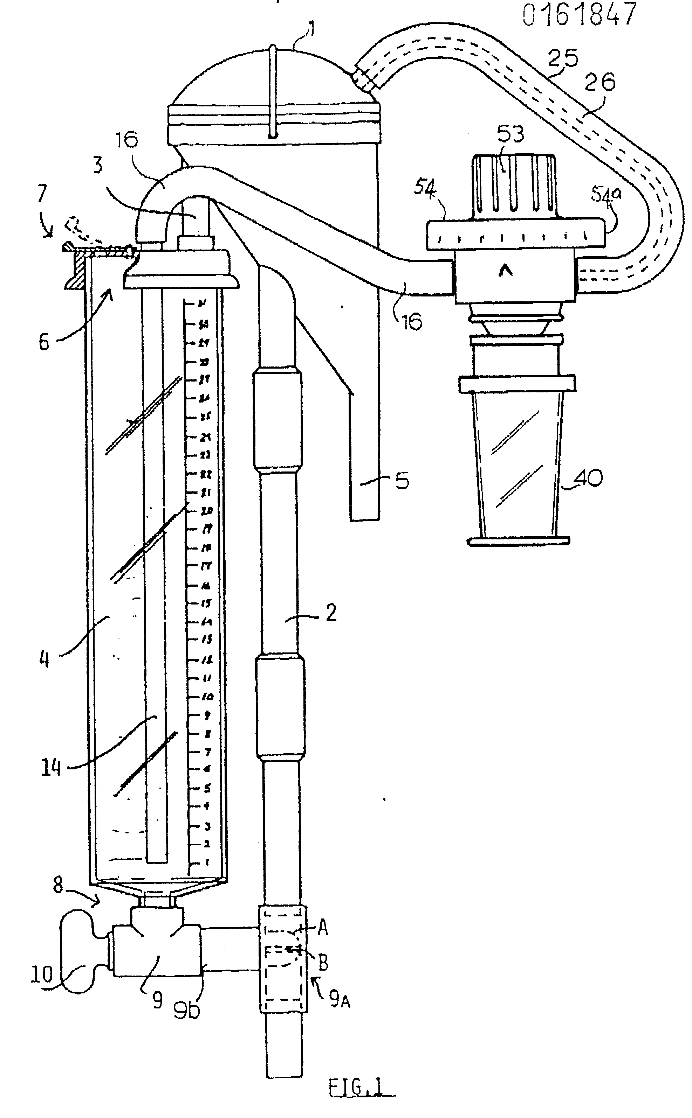

We refer firstly to Figure 1 of the accompanying drawings.

The present invention includes a metering arrangement 1 such as that described for example in United States patent specification No. 3,349,617. This is however by way of example only, and the present invention and the subject matter thereof, can be used in conjunction with any appropriate metering arrangement.

A milk inlet line 2 is provided leading into the metering arrangement 1 leading from the milking cups associated with milking machinery (not shown), which draw milk from a cow. Milk is drawn into the metering arrangement 1 by vacuum and a proportion of the milk passes from the metering arrangement 1 through a transfer means, connection tube or bore 3, and into a sampling chamber 4. A valve (not shown), such as for example a flap valve, is provided at the connection between the outlet passage 3 and the chamber 4. The remainder of the milk from the meter 1 exits through the outlet 5 into appropriate milk collection or storage mans (not shown).

The chamber 4 is preferably in the form of an elongate container or tubular vial, which is provided with appropriate markings or calibrations on the exterior thereof, so that a clear indication can be obtained as to the amount of liquid therein. Preferably the chamber 4 if constructed of an at least substantially transparent plastic or gloss material. Other materials can however be used.

At the upper end of the chamber 4 is an air inlet 6. This can be in the form of one or more holes or bores covered by an appropriate cover or valve 7. The valve 7 may be a flap or plate valve (for example of a flexible and/or resilient material), which is preferably biased into a closed position against the inlet 6. If however it is desired to allow for the entry of air (as will be described hereinafter), the valve 7 is merely lifted against its natural bias, to allow for the entry of such air. It should be appreciated that any form of the appropriate valving can be used.

At the lower end of the main chamber 4 there is provided an outlet 8 which is in turn provided with appropriate valving 9.

The milk inlet line 2 and the main chamber 4 are connected one to the other through the valving 9. The valving 9 includes a tubular connection linking the milk inlet 2 and the chamber 4.

The particular advantage of this connection and valving (to be described further hereinafter) is that it allows cleaning of the equipment in a particularly efficient and straight forward manner as compared with the methods of cleaning used up until this time. In particular it allows for what is commonly known as "CIP", that is; cleaning in place.

Up until this time, when milking had finished and it was desired to clean the equipment (for example with water or water and a suitable surfactant or cleaning material) it was necessary to change around the equipment to allow for a counter-flow against that which normally occured. Sometimes it was necessary to disassemble the equipment for satisfactory cleaning. By providing an arrangement according to the present invention, the valving 9 can be operated (such as by movement of the toggle handle 10) which allows for a complete "flow through" of cleaning liquid.

The valving 9 includes an elongate tubular shaft 9b which has a basically flat valve 9a at the end thereof, the flat valve 9a preferably being constructed of a flexible or semi-flexible material, which extends into (and is capable of controlling flow through) the inlet pipe 2. On actuation (rotation) of the toggle 10, the shafe 9b and valve 9a can be moved into a position substantially as shown at "A" in Figure 1 of the drawings, wherein connection between the valving 9 and the main chamber 4 is closed, but wherein the flap valve 9a is in a substantially vertical or upright position in which it does not obstruct (or provides minimal obstruction to) the flow of milk through the inlet line 2.

In a further form of the invention, the valve 9 can be actuated into a further position (such as by rotation of the toggle handle 10) which opens valving between the valve 9 and the main chamber 4, in turn moving the flap valve 9a into a substantially horizontal or flat position (in which it is substantially transverse to the longitudinal axis of the inlet line 2) and in which it at least partially obstructs the flow of liquid through the milk line 2. This is shown in dotted lines "B" in Figure 1 of the drawings.

When milking is in progress, the toggle 10 and valve 9 are actuated so that valving between the chamber 4 and the valve 9 is closed, and so the flap valve 9a is essentially in the position "A"; this closes the valving between the main chamber 4 and the valve 9 (and indeed the milk line 2) so that the milk flows through the milk line 2 and into the metering device 1. Once milking has ceased, and it is desired to carry out cleaning, appropriate connections are made to allow for the flow of water and/or a surfactant and cleaning liquid through the inlet line 2. The toggle 10 and valve 9 are then moved into a further position in which the flap valve 9a is in an upright or substantially vertical position substantially shown as "B" in Figure 1 of the drawings. The movement of the toggle 10 also opens the valving between the main chamber 4 and 9, and connects also the milk line 2 to the main chamber 4.

Due to the location of the flap valve, the flow of liquid through the milk line 2 will be substantially diverted through the valving 9 and up into the chamber 4. At the same time, however, due to the said flexibility/resilience of the valve 9a, a proportion of the liquid will also be caused to pass up the milk line 2 thus allowing for a "flow through" of cleaning liquid to allow for complete cleaning in place. By way of example only, in one form of the invention about 80% of the cleaning liquid can pass through the valve 9 into the chamber 4, where as, about 20% will pass up the inlet line 2.

If desired, the valving 9 can be provided with a number of different positions, allowing for differing rates of flow through inlet line 2 and/or into the main chamber 4.

In another form of the invention, and referring further to Figure 1 of the accompanying drawings, while the main chamber 4 is receiving a sample of milk from the meter 1, (in turn from the milk line 2), this is often a relatively large amount of milk. Thus is often required or desirable to have a smaller but still representative amount for the purpose of sampling. Accordingly, it is advantageous to provide a further sampling arrangement which provides for an effective "sub-sample" from the main chamber 4, but which is still proportional to and representative of, the milk in the main chamber 4.

For this purpose, a sub-sampler arrangement 12 is provided.

In order to pass the milk from the main chamber 4 into the sub-sampler 12, outlet line 14 is provided with the chamber 4, being substantially elongate in formation and extending through a sealed recess in the top of the main chamber 4, thereafter being connected to an inlet line leading to the sampler 12. The outlet line 14 is elongate and constructed of any appropriate material such as for example stainless steel and is spaced apart from the bottom of the main chamber 2.

Thus, in use milk is exited from the chamber 4 through the outlet line 14, and through the outlet line 15 which is connected to the inlet 20 of the sub-sampler 12. An outlet line 25 from the sub-sampler 12, leads from the outlet 21 back to the meter 1, to which it is connected and in communication. Preferably, the outlet line 25 connects to an upper portion 1a of the meter 1. This is not however essential.

Thus, when it is desired to obtain a proportional representation of the milk from the main chamber 4, it is necessary to pass the milk in that chamber through and into the sub-sampler. The meter 1 is generally under vacuum, and thus, the valve 7 associated with the air inlet 6 is opened or lifted (so as to communicate with atmosphere). This allows air into the main chamber 4 which will inturn draw losed the valve between the entry pipe 3 from the meter 1, and the main chamber 4. The entry of air into the chamber 4 causes milk within the chamber 4 to pass up through the outlet pipe 14, through the inlet line 16 and into the sub-sampler 12.

This then is an efficient and straight forward way of transferring milk from the main chamber 4 into the sub-sampler 12.

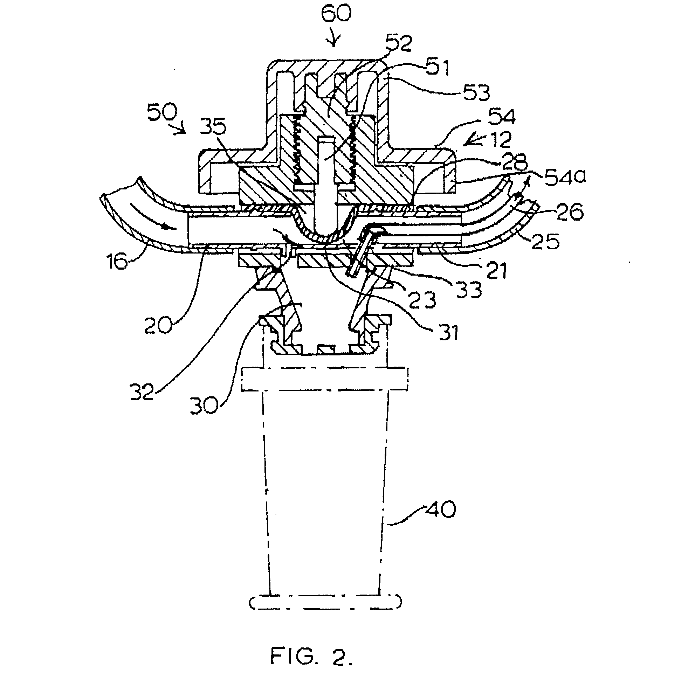

Referring now to Figure 2 of the accompanying drawings, this shows a sectional view of a sub-sampler 12 according to one form of the invention. The sub-sampler 12 has a housing 60 which includes a chamber 30 and a substantially transverse bore 31, through which a liquid flow line 23 (to be described hereinafter) can pass. A liquid inlet 20 extends into the housing and a liuqid outlet 21 extends outwardly of the housing. In use, a liquid inlet line 16 (such as from the chamber 4), is connected to the inlet 20. An elongate outlet line 25 can be attached to the outlet 21, so as for example to extend to a connection with the meter 1. The connection lines 16 and 25 are preferably constructed of a flexible material. This is by way of example only however. The liquid flow line 23 extends between the inlet 20 and the outlet 21 so as to extend through a transverse bore 31 of the housing. Preferably a bore, recess or scallop 35 is formed in an upper surface of the liquid flow line, substantially medially thereof, and an elongate, tubular sleeve 28, of a flexible and/or resilient material is located over and about at least part of the flow line 23 so as to also pass over and about the recess, bore or scallop 35 therein. Annular ribs or ridges can be provided about the outer surface of the flow line 23, so as to hold the flexible and/or resilient sleeve 28 in position.

The housing is provided with the lower chamber 30 which is in communication with the liquid flow line 23. The chamber 30 is substantially hollow, and is provided with means to enable it to releasably locate or engage with a sample vial or container 40, to collect a sample from liquid passing through the sub-sampler 12 (as will be described hereinafter).

One or more holes or connection lines 32 extend from the flow line 23 into the chamber 30. For example in one form of the invention a slot or hole 23 can be provided in the liquid flow line, and a tube engaged therewithin, so that a tubular connection is provided between the liquid flow line 23 and the chamber 30 inwardly of the inlet 20. Preferably, an air outlet 33 is provided extending from the chamber 30 into the liquid flow line 23, spaced inwardly of the outlet 21. Preferably a tubular connection is provided as will be described hereinafter.

In use therefore, liquid flowing through the liquid flow line 23 (having entered through the inlet line 16) will pass through the liquid flow line 23 and a proportion of the liquid is able to pass into the chamber 30 through the liquid inlet 32. The liquid then passes into the sample vial or container 40 releasably connected to the chamber 30. Excess air carried into the chamber 30 by the liquid, exits through the air outlet 33 as referred to hereinbefore.

The sub-sampler 12 provides means for controlling the flow of liquid through the liquid flow line 23, and thus controlling the amount of liquid that passes through the liquid inlet 32, into the chamber 30 and any vial or container releasably engaged therewith. In the form of the invention shown in Figure 2 of the drawings, the control means 50 is in the form of a restriction member, which is an elongate plunger 51, which is mounted adjacent and above the liquid flow line 23, and in particular immediately above the bore or scallop 35 as covered by the flexible and/or resilient sleeve 28. The plunger 51 is preferably mounted within appropriate mounting and actuating means, and is preferably spring biased into a position away from the liquid flow line 23. The plunger 51 is preferably axially mounted within an appropriate boss 52, about which actuating means may be engaged by screw thread and the like, so as to be capable of being rotated, such rotation causing the plunger 51 to move axially downwardly, against spring bias, and to exert influence on the flexible and/or resilient sleeve 28 extending over the bore or scallop 35. A continued rotation of the actuating means 50 and downward axial movement of the plunger 51 causes the flexible and/or resilient sleeve 28 to move through the bore or scallop 35 and into the path of the flow line, thus restricting the flow of liquid therethrough. Thus, the more the plunger 51 is moved downwardly, the more restricted is the flow of liquid through the flow line 23, and thus the more liquid which is diverted through the liquid inlet 32, into the chamber 30 (and thus into any vial or container 40 releasably engaged therewith). As will be appreciated from Figure 2 of the drawings, the plunger 51 is shown in a position in which it

substantially closes off the flow of liquid through the flow line 23, and thereby diverts a substantial proportion of the liquid through the liquid inlet 32, into the chamber 40.

The actuating means 50 of the present invention are in the form of a fixed boss member 52, and an outer cap, a central boss portion 53 and a radially extending flange 54, having a downwardly depending skirt 54 at its outer periphery. This engages (such as by screw thread) for rotational movement about and relative to, the boss 52.

In a preferred form of the invention, the outer surface of the skirt 54 can be provided with calibrations or graduations thereon, and a central marker or arrow, can be provided on the main body portion of the housing (such as in a position substantially intermediate the liquid flow line 23, and substantially aligned with location of said plunger 51).

In use therefore, if it is desired to obtain a sample of liquid proportional to the amount of liquid in the chamber 4, (as shown in Figure 1 of the accompanying drawings), the actuating means 50 is rotated so that the graduation or calibration on the skirt thereof, as positioned by the pointer or marker, on the body of the housing, corresponds to the level of liquid within the chamber (as shown by markings or graduations on the chamber 4). This will then determine the degree to which the flow of liquid is restricted as it passes through the liquid flow line 23, and thus provides a sample from the chamber 4, which is substantially proportional and representative of the liquid in the chamber 4.

While the invention has been described by way of example only, to the actuating means being in the form of a rotatable control means, which causes axial longitudinal movement of the plunger 51, towards and away from the flexible/resilient sleeve 28 passing about the liquid flow line 23, it should be appreciated that other means can be used to move the plunger or some similar mechanism, inwardly and outwardly of the liquid flow line. Also, other means can be provided for restricting the flow of liquid passing through the liquid flow line, such as for example ventural arrangements; variable valving arrangements and the like. The arrangement described with reference to Figure 2 of the accompanying drawings is by way of example only.

Referring now to the air outlet 33, extending from the chamber 30 into the outlet 21. Preferably an elongate tubular outlet extends from the chamber 30 into the liquid flow line 23, inwardly of the outlet 21. As indicated hereinbefore, a liquid outline 25 is connected to the outlet 21 and preferably passes into the meter 1, so that in use the liquid passing through the liquid flow line, exits from the outlet 21 then passes through the outlet line 25 back into the meter 1, where at least the majority thereof will pass through the outlet 5 into an appropriate liquid collection or storage means. It is possible however that some of the liquid might again pass through the meter 1 into the chamber 4 to be recirculated.

An air outlet line 33 allows air which passes into the chamber with liquid, to be exited from the chamber 30, as the presence of such air would detract from the efficiency and operation of the invention.

In one form of the invention the air outlet 33 can merely open the air into the liquid flow line 23 and the outlet line 25, where it will join liquid and pass back into the meter 1. In a preferred form of the invention however, as shown in Figures 1 and 2 of the accompanying drawings, an elongate tube 26 of relatively small diameter, is connected to the air outlet 33 and passes through the liquid outlet line 25, substantially concentrically, so as to exit also into the meter 1. The air from the air outlet 33 therefore travels separately (from the liquid) through the liquid outlet 25, to be passed into the meter 1. This has been found to be particularly efficient in use, as it avoids the passage of air directly into the liquid flow line 23 and liquid outlet line 25, which could cause turbulence, back flow, and generally detract from the efficiency of operation.

It should be appreciated that modifications and improvements may be made to this invention without departing from the scope of or spirit thereof.

WE CLAIM:

1. A liquid metering arrangement including a liquid meter (1); a liquid inlet line (2) communicating with said meter (1); a liquid sample chamber (4) being in communication with said meter (1); said liquid inlet line (2) and said sample chamber (4) being connected to allow for the flow of liquid therebetween ; valve means (9) being provided in association with said connection, such that on movement of said valve (9) to at least a first position, flow of liquid from the inlet line (2), through said connection (9) and into the chamber (4), is substantially avoided; movement of said valve (9) to at least a second position allowing at least a proportion of liquid passing through the inlet line (2), to be diverted through said connection (9) and into said chamber (14).

2. An arrangement as claimed in claim 1, wherein movement of said valve (9) to at least a second position, allows an amount of liquid to continue to pass through said inlet line (4), to said meter (1).

3. An arrangement as claimed in claim 1 or claim 2, wherein the sample chamber (4) is substantially elongate in formation, the meter (1) being located and in communication therewith, adjacent an upper end thereof; the connection (9) between said sample chamber (4) and said inlet line (2) being adjacent the bottom of said sample chamber (4).

4. A liquid metering arrangement including a liquid meter (1) and at least one sample chamber (4) in communication therewith; transfer means (3) being provided to allow for the passage of liquid from said meter (1) into said sample chamber (4); valve means being provided in association with said transfer means (3); an air inlet (6) being provided adjacent an upper portion of the sample chamber (4) and having valve means (7) associated therewith; a liquid outlet pipe (14) being provided within said sample chamber (4), and extending out of an upper portion thereof, so as to be connectable to a sub-sampler (12); the arrangement being such that on an amount of liquid being present within said sample chamber (4), said valving (7) associated with said air inlet (6) into said sample chamber (4) is opened allowing an inlet of air, thereby causing closure of said valving associated with the liquid transfer means (3) and further causing liquid within the sample chamber (4) to pass through the liquid outlet pipe (14) to said sub-sampler (12).

5. A liquid metering arrangement including a liquid meter (1) and at least one sample chamber (4) in communication therewith; liquid supply means (2) leading to said meter (1), the arrangement being such that at least a proportion of said liquid passes into said chamber (4); a liquid inlet line (16) leading from an outlet of said chamber (4) to a sub-sampler (12); means being provided to cause the flow of liquid from said chamber (4) through said inlet line (16) and into said sub-sampler (12); said sub-sampler (12) including a housing (60) having an inlet (20) and an outlet (21); said liquid inlet line (16) being connected to said inlet (20); a liquid outlet line (25) being connected to said outlet (21) and thereafter extending to said meter (1) so as to be in communication therewith; a liquid flow line (23) being provided within said housing (50), and connecting said inlet (20) and outlet (21); a chamber (30) being provided and being in communication with said liquid flow line (23) such that a proportion of liquid passing through said liquid flow line (23) is able to pass into said chamber (30); said chamber (30) being adapted so as to releaseably engage and locate a sample vial or container (40).

6. An arrangement as claimed in claim 5, wherein said sub-sampler (12) includes control means (50) to restrict passage of liquid through said liquid flow line (23).

7. An arrangement as claimed in claim 5, wherein an air outlet (33) leads from said chamber (30) into said liquid flow line (23).

8. An arrangement as claimed in claim 7, wherein said control means (50) are provided substantially intermediate the inlet (33) from said liquid flow line (23) into said chamber (30), and said air outlet (21) from said chamber (30) into said liquid flow line (23).

9. An arrangement as claimed in claim 6, wherein the control means (50) is mounted outwardly of said liquid flow line (23); actuating means being associated therewith so that on actuation thereof, restriction means (51) is caused to move inwardly of said liquid flow line (23), so as to be capable of restricting

0161847

- 22 -

the flow of liquid therethrough.

10. An arrangement as claimed in claim 9, wherein the liquid flow line (23) is provided with at least one bore or recess (35) therein; a substantially flexible and resilient sleeve (28) extending about at least part of said liquid flow line (23) and over said bore or recess (35); said restriction means including an elongate plunger (51) mounted adjacent and over said bore or recess (35); the arrangement being such that on actuation of said actuating means, said elongate plunger (51) is caused to move substantially inwardly and axially against said sleeve (28) and through said bore or recess (35) so as to form an at least partial restriction in said liquid flow line (23).

0161847

11. An arrangement as claimed in claim 9, wherein said control means (50) is in the form of a rotatable grip member having a substantially central boss portion (53) and a radially extending flange (54) with a peripheral, downwardly depending skirt (54a); said skirt (54a) being provided with a plurality of markings or calibrations thereon; at least one marking being provided on the body portion of said sub-sampler (12), at a position substantially aligned with the location of said elongate plunger (51).

12. An arrangement as claimed in claim 5, wherein a tubular inlet line 32 extends from said liquid flow line 23 into said chamber (30) at a position inwardly of said inlet (20); a tubular air outlet line (33) leading from said chamber (30) into said flow line (23), at a position spaced apart inwardly from said outlet (21).

13. An arrangement as claimed in claim 6, wherein said air outlet line (21) is in the form of an elongate tube extending from said chamber (30), through said liquid flow line (23) and said liquid outlet line (25) so as to be in communication with said meter (1); the diameter of said tubular air outlet (26) being less than the diameter of said liquid outlet line (25) leading from the outlet (21) of said housing to said meter (1).

14. A sample arrangement for obtaining a sample of liquid from a liquid flow, including a housing (60); liquid inlet and outlet means (20,21) being provided; a liquid flow line (23) extending therebetween and within said

0161847

housing (60); a chamber (30) being provided and adapted so as to releasably engage a sample vial or container (40); said liquid flow line (23) being in communication with said chamber (30) such that a proportion of liquid passing through said liquid flow line (23) is able to pass into said chamber (40).

1/2

0161847

FIG.1

2/2

0161847

FIG. 2.